# Europäisches Patentamt

## European Patent Office

## Office européen des brevets

(19)

(11) Veröffentlichungsnummer: **0 177 912**
**B1**

(12) # EUROPÄISCHE PATENTSCHRIFT

(45) Veröffentlichungstag der Patentschrift:
16.03.88

(51) Int. Cl.⁴: **C 07 C 31/20,** C 07 C 29/17

(21) Anmeldenummer: **85112645.8**

(22) Anmeldetag: **05.10.85**

(54) **Verfahren zur Herstellung von Alkandiolen.**

(30) Priorität: **12.10.84 DE 3437429**
**01.12.84 DE 3443966**

(43) Veröffentlichungstag der Anmeldung:
**16.04.86 Patentblatt 86/16**

(45) Bekanntmachung des Hinweises auf die Patenterteilung:
**16.03.88 Patentblatt 88/11**

(84) Benannte Vertragsstaaten:
**BE CH DE FR GB IT LI NL**

(56) Entgegenhaltungen:
**DE-A-2 004 611**
**DE-A-2 145 297**
**DE-A-2 926 641**
**DE-B-1 668 187**
**DE-C-890 944**
**US-A-3 950 441**
**US-A-4 371 723**
**US-A-4 438 285**

(73) Patentinhaber: **BASF Aktiengesellschaft, Carl-Bosch- Strasse 38, D-6700 Ludwigshafen (DE)**

(72) Erfinder: **Mueller, Herbert, Dr., Carostrasse 53, D-6710 Frankenthal (DE)**
Erfinder: **Reiss, Wolfgang, Dr., Bannwasserstrasse 70, D-6700 Ludwigshafen (DE)**

**Beschreibung**

Diese Erfindung betrifft ein Verfahren zur Herstellung von Alkandiole, durch Hydrierung von Alkindiolen.

Alkandiole, wie Butandiol-1,4, lassen sich durch katalytische Hydrierung von Alkindiolen, wie Butindiol-1,4, herstellen. Bei diesem z. B. in der DE-PS-890 944 oder der US-PS-4 153 578 beschriebenen Verfahren verwendet man z. B. Nickel- oder Kobaltkatalysatoren, die entweder als Raney-Metalle oder als Trägerkatalysatoren eingesetzt werden. Auch Trägerkatalysatoren, die neben Nickel noch Kupfer, Aluminium und Mangan enthalten, sind schon für die Hydrierung von Acetylenalkoholen vorgeschlagen worden (s. DE-OS-2 145 297, US-PS-3 449 445 und DE-OS-2 004 611).

In der DE-OS-2 536 273 wird ein festangeordneter Nickelkatalysator beschrieben, der zusätzlich noch die Elemente Kupfer, Mangan und Molybdän enthält. Mit diesem Katalysator konnten zwar die Standzeiten der herkömmlichen Katalysatoren bei der Hydrierung von Butindiol-1,4 zu Butandiol-1,4 verbessert werden. Die Resultate befriedigen jedoch noch nicht völlig. Um ein Butandiol zu erhalten, das hohen Reinheitsanforderungen entspricht, müssen verhältnismäßig hohe Wasserstoffdrücke und Temperaturen gewählt werden, da es nur so gelingt, die sich bei vielen Anwendungen nachteilig auswirkenden Carbonylverbindungen und deren Derivate soweit reduzierend zu entfernen, daß ihr schädlicher Einfluß toleriert werden kann. Eine derartige Verschärfung der Reaktionsbedingungen fördert jedoch die unerwünschte Bildung von Butanol und vermindert die Ausbeute. Gleichzeitig erhöht sich der Anfall des unerwünschten Nebenproduktes 2-Methylbutandiol (s. US-PS-4 153 578). Schließlich macht es auch Schwierigkeiten, den die aktiven Metalle enthaltenden Katalysator reproduzierbar mit gleicher Aktivität und Lebensdauer herzustellen.

Für die Hydrierung der Alkinole werden die Katalysatoren in suspendierter oder fest angeordneter Form eingesetzt. Man unterscheidet die einstufige Hydrierung, bei der die Hydrierung in einem einmaligen Durchgang durch einen Reaktor erfolgt, und die zweistufige Arbeitsweise. Beim Zweistufen-Prozeß (US-PS-4 153 578) wird in der ersten Stufe bei niederen Drücken und tiefer Temperatur unvollständig hydriert. Ein Teil des Alkinols oder der im Zulauf enthaltenen Carbonylverbindungen bleiben unumgesetzt. Erst in der Nachreaktionszone, in der bei Wasserstoffdrücken über 200 bar und Temperaturen über 100° C gearbeitet wird, findet die vollständige Hydrierung statt.

Da Butandiol-1,4 in großen Mengen technisch verwendet wird, hat die Hydrierung von Butin-2-diol-1,4 besonderes Interesse gefunden. Grundsätzlich läßt sich die Reaktion bei Drücken bis 400 bar durchführen. Drücke unter 150 bar ergeben ein Butandiol, dessen Reinheit den gestiegenen Ansprüchen bei der Verwendung nicht entspricht. Man hydriert deshalb bei Drücken zwischen 200 und 350 bar. Niedere Drücke sowie hohe Temperaturen bei der Hydrierung begünstigen die Butanol-Bildung. Hohe Drücke verteuern die Hydrierapparatur. Die Hydriertemperaturen liegen bei den bekannten technischen Verfahren an festangeordneten Katalysatoren zwischen 70° C am Eingang und 160° C am Ausgang der Reaktoren. Anfangstemperaturen unterhalb von 70° C gelten als unwirtschaftlich, da zu lange Katalysatorstrecken zum Erreichen der "Anspringtemperatur" benötigt werden, während Temperaturen über 160° C die Standzeit der Trägerkatalysatoren, die ohnehin verhältnismäßig kurz ist, erheblich verschlechtern können. Die Hydrierung bei hoher Temperatur liefert zwar ein qualitativ gutes Butandiol, die Ausbeute geht aber mit steigender Temperatur zurück. Es ist allgemein bekannt und betrieblich ausgeübte Praxis, die Hydriertemperaturen nicht über 150° C steigen zu lassen, da dann eine überproportional gesteigerte Butanol-Bildung auf Kosten von Butandiol stattfindet (s. Ann. der Chemie, Bd. 596 (1955), S. 41 und 42 und DE-OS-2 004 611, S. 5, Abs. 3). Höhere Temperaturen sollten auch vermieden werden. weil sie wegen der in den zu hydrierenden Rohlösungen vorhandenen Alkalisalze die Favorsikii-Spaltung begünstigen (s. Houben-Weyl, Methoden der organischen Chemie, Band 5, Teil 2a, S. 650).

Eine besonders schwierige Aufgabe bei der Hydrierung von Butindiol nach dem Reppe-Prozeß zu Butandiol ist die Entfernung von Carbonyl-Verbindungen bzw. deren Acetale. Butindiol-Lösungen enthalten von der Herstellung her stets nicht zu vernachlässigende Mengen an Formaldehyd, durch die die Hydrierung des Butindiols stark gestört oder unerwünschte Nebenprodukte gebildet werden. Es wurde deshalb schon vorgeschlagen, diesen Formaldehyd durch Destillation vor der Hydrierung aus den Roh-Butindiol-Lösungen zu entfernen (vgl. US-PS-3 449 445, Spalte 2, Zeilen 57 bis 60 und US-PS-2 993 078. Um den schädlichen Einfluß des Formaldehyds zu vermeiden, wurde sogar in der US-PS-4 180 687 der Vorschlag gemacht, den Formaldehyd in der Butindiol-Roh-Lösung durch starkes Alkali zu polymerisieren, um ihn so für die Hydrierung unschädlich zu machen. Außer der vergiftenden Wirkung auf den Hydrierkatalysator bewirkt der Formaldehyd, daß aus Butindiol bei der Hydrierung 2-Methylbutandiol-1,4 gebildet wird. Dieses Produkt ist im Rein-Butandiol sehr unerwünscht und läßt sich außerdem destillativ kaum vom gewünschten Butandiol-1,4 abtrennen. Deshalb wird in der US-PS-4 153 578 vorgeschlagen, die Butindiol-Lösung zunächst bei niederen Drücken und niederen Temperaturen weitgehend zu hydrieren und dann in einer zweiten Stufe an einem speziellen molybdänhaltigen Raney-Nickel zu Ende zu hydrieren. Dadurch gelingt es, den Methylbutandiol-Gehalt im Butandiol von normalerweise 1,5 bis 2 % auf bestenfalls 0,3 bis 0,6 % zu reduzieren. Ein solches Verfahren ist verfahrenstechnisch aufwendig und mit erheblichen Kosten verbunden.

Ein schwerwiegendes Problem bei der Herstellung von Butandiol aus Butindiol-Lösung haben alle Hydrierverfahren nicht lösen können. Bekanntlich enthalten Butindiol-Lösungen von der Herstellung her nicht nur freien Formaldehyd sondern auch mehr als 0,2 Gew.-% Polyoximethylene. Diese können die aktiven Katalysatoroberflächen belegen und somit die Hydrierwirkung beeinträchtigen oder aber als wertloser

# 0 177 912

Rückstand erscheinen, dessen Beseitigung bei der destillativen Aufarbeitung der Butandiol-Roh-Lösung Kosten verursacht. Es hat sich auch gezeigt, daß die niederen Polyoximethylene im Rein-Butandiol als Verunreinigungen enthalten sein können, wenn die destillative Aufarbeitung nicht mit einem sehr hohen technischen Aufwand durchgeführt wird. Solches Butandiol z. B. ist ungeeignet, um für die Herstellung von Tetrahydrofuran (durch Dehydratisierung aus Butandiol) eingesetzt zu werden. Die Polyoximethylene reduzieren nämlich die Aktivität der Dehydratisierungskatalysatoren.

Es bestand die Aufgabe, für die Hydrierung von Acetylenalkoholen ein Verfahren zu finden, das nicht nur einen hohen Durchsatz ermöglicht, sondern auch den Nachteil hoher Katalysatorkosten, die durch die begrenzte Standzeit von Trägerkatalysatoren oder die hohen Herstellkosten von Vollkatalysatoren verursacht werden, vermeidet. Gleichzeitig sollte es sehr reine Alkandiole liefern und die Hydrierung von Butin-2-diol-1,4 mit 99 bis 100 % Ausbeute ermöglichen. Darüberhinaus sollte das Verfahren die Möglichkeit erschließen, im Butin-2-diol-1,4 vorhandene Nebenprodukte, wie Hydroxibutyraldehyd, davon abgeleitete Acetale und Acetale des Butindiols in Butandiol-1,4 zu überführen. Weiter war an das Hydrierverfahren die Aufgabe zu stellen, den im Zulauf der Alkindiol-Lösungen vorhandenen Formaldehyd und dessen Polymere in Methanol zu überführen. Die Hydrierung der Formaldehyd-Polymere ist nämlich insofern von Bedeutung, als sie zur Entlastung der Reindestillation des Butandiols einen entscheidenden Beitrag liefert und durch die Nebenausbeute Methanol die Wirtschaftlichkeit des Verfahrens signifikant erhöht. Gleichzeitig war von dem neuen Hydrierverfahren zu fordern, daß es sehr reine Alkandiole liefert, die auf dem Hochpolymerensektor oder zur Herstellung von cyclischen Ethern, wie Tetrahydrofuran eingesetzt werden können. So ist z. B. die Herstellung eines Tetrahydrofurans erwünscht, das ohne zusätzliche Reinigung geeignet ist, zu Polytetrahydrofuran polymerisiert werden zu können.

Nach dem neuen Verfahren, das diese Aufgabe erfüllt, stellt man Alkandiole durch katalytische Hydrierung von Alkindiolen in wäßrigem Medium und in Gegenwart eines Hydrierkatalysators bei Drücken über 20 bar Wasserstoffpartialdruck dadurch her, daß man die Hydrierung bei Temperaturen von über 200° C vornimmt.

Das erfindungsgemäße Verfahren zeichnet sich insbesondere dadurch aus, daß es die Hydrierung von Butin-2-diol-1,4 zu Butandiol-1,4 in praktisch quantitativer Ausbeute ermöglicht und dabei ein Endprodukt von extrem hoher Reinheit liefert. Das Butandiol ist frei von Carbonylfunktionen oder Spuren von olefinisch ungesättigten Verbindungen. Der Gehalt an Methylbutandiol liegt bereits beim unmittelbaren Verfahrensprodukt bei unter 0,1 Gew.-%. Der Rückstandswert dieser rohen Butandiol-Lösung liegt unter 0, 2 Gew.-%, während er bei den rohen Butandiol-Lösungen, die man bei den bekannten Hydrierverfahren erhält, zwischen 1 und 2 Gew.-% liegt. Das neue Verfahren zeichnet sich durch eine erhöhte Wirtschaftlichkeit aus, weil es die Gewinnung der bei der Reaktion entstehenden Hydrierwärme bei einem hohen Temperaturniveau ermöglicht.

Als Alkindiole werden für das Verfahren der Erfindung z. B. solche mit 3 bis 6 C-Atomen, wie Butin- 2-diol-1,4 oder Hexin-3-diol-2,5 eingesetzt. Die Hydrierung von Butin-2-diol-1, 4 zum Butandiol-1,4 ist von besonderem technischen Interesse.

Nach dem erfindungsgemäßen Verfahren hydriert man die Alkindiole in wäßrigem Medium. In den wäßrigen Ausgangsgemischen sind die Alkindiole im allgemeinen mit einer Konzentration von 10 bis 60 Gew.-% gelöst. Die wäßrigen Gemische enthalten daneben meist Verunreinigungen in einer Konzentration von 2 bis 8 Gew.-%, z. B. Aldehyde, wie Formaldehyd und dessen Polymere, Acetale oder auch monofunktionelle Alkinole, wie Propin-2-ol-1. Sie können außerdem z. B. 10 bis 50 Gew.-% des gesättigten Diols enthalten, das durch Hydrierung des Alkindiols entsteht. Wäßrige Gemische der genannten Art werden z. B. großtechnisch durch Umsetzung von Acetylen mit wäßrigem Formaldehyd erhalten. Man hydriert vorzugsweise bei einem pH-Wert der wäßrigen Ausgangslösung von größer als 6. Falls die Roh-Butindiol-Lösungen einen niedrigeren pH-Wert aufweisen, können sie, z. B. durch Alkalizugabe, auf einen pH-Wert von größer als 6, vorzugsweise auf pH-Werte von zwischen 6,5 und 9, eingestellt werden.

Für das erfindungsgemäße Verfahren sind alle gebräuchlichen Hydrierkatalysatoren geeignet, z. B. die für die katalytische Hydrierung geeigneten Metalle, wie Platin, Palladium, Ruthenium, Nickel, Cobalt oder Kupfer. Bevorzugt werden Katalysatoren, die nur Nickel als katalytisch wirkende Komponente enthalten. Besonders geeignet ist z. B. Raney-Nickel oder Nickel, das durch den Zerfall von Nickel-0-Komplexen oder Nickelformiat gebildet wird. Das aktive Metall kann auch auf einem Träger niedergeschlagen sein. Es sind auch Katalysatoren für das Verfahren einsetzbar, die mehrere der bekannten Hydriermetalle als wirksame Komponenten enthalten. Auch der für die Butindiol-Hydrierung üblicherweise eingesetzte Katalysator aus Nickel, Kupfer und Mangan auf Kieselsäure oder aluminiumoxidhaltigen Trägern ist geeignet. Die Katalysatoren werden vorzugsweise in suspendierter Form eingesetzt. Sie werden z. B. dem wäßrigem Medium vor der Hydrierung in feinverteilter Form zugegeben. Man kann anstelle des metallischen Nickels auch wasserlösliche Nickelsalze einer organischen Säure mit mindestens 2 C-Atomen verwenden. Wasserlösliche Nickelsalze einer organischen Säure mit mindestens 2 C-Atomen, sind z. B. Nickelacetat, Nickelpropionat und Nickelbutyrat. Mit Nickelacetat und Nickelpropionat erhält man besonders vorteilhafte Ergebnisse. Bei dieser Arbeitsweise gibt man zu dem zu hydrierenden wäßrigen Gemisch so viel Nickelsalz, daß es Nickel in einer Konzentration von 0,01 bis 0,2 Gew.-% enthält. Die bevorzugte Konzentration an Nickel beträgt 0,05 bis 0,15 Gew. %, bezogen auf die wäßrige Alkindiol-Lösung. Höhere Konzentrationen sind für die Umsetzung unschädlich, verringern aber die Wirtschaftlichkeit des Verfahrens.

Man hydriert bei Temperaturen von über 200° C, wie bei 210 bis 280° C, vorzugsweise bei 230 bis 260° C und z. B. bei Drücken zwischen 20 und 300 bar, vorzugsweise zwischen 50 und 250 bar. Drücke, die über den bevorzugten Bereich hinausgehen, bringen gemessen am Aufwand keine merklichen Vorteile. So läßt sich z. B.

3

aus Butindiol an 1 Gew.-% Raney-Nickel in einer Stufe bei Temperaturen um 250°C und einem Druck von 250 bar ein Butandiol höchster Reinheit mit praktisch quantitativer Selektivität herstellen. Die Konzentration des Hydrierkatalysators richtet sich nach der gewünschten Reaktionsgeschwindigkeit bei der gewählten Reaktionstemperatur. Sie liegt im allgemeinen mit z. B. 0,05 bis 5 Gew.-% wesentlich niedriger als bei der sonst geübten Hydrierarbeitsweise. Trägerfreie Katalysatoren werden bevorzugt, da sie sich durch Dekantieren oder Magnet-und Schichtenfiltration besonders leicht und vollständig abtrennen und auch-rückführen lassen.

Die Hydrierung kann in einfachen Reaktionsapparaten, wie Blasensäulen, gerührten Reaktionsbehältern oder Umlaufreaktoren, die z. B. durch Düsen für einen ausreichenden Stoffaustausch und zur Durchmischung des Reaktionsgemisches sorgen, durchgeführt werden.

Bei kontinuierlicher Verfahrensführung, die man z. B. in einem gerührten Reaktor oder einem Umlaufreaktor durchführt, wird die Hydrierwärme zweckmäßigerweise dazu verwendet, den Frischzulauf z. B. auf eine Eingangstemperatur von 160 bis 180°C aufzuheizen, ehe er in den Reaktor eintritt. Das Aufheizen erfolgt z. B. über einen außenliegenden Wärmetauscher, der vom rückgeführten Reaktionsgemisch durchströmt wird. In dem Maße, in dem die Hydrierung längs der Reaktionszone fortschreitet, erhitzt sich das Reaktionsgemisch auf über 200°C. Die Endtemperatur wird in diesem Falle überwiegend von der Menge des Frischzulaufs und der z. B. für die Dampfgewinnung verwendeten Menge der Hydrierwärme bestimmt.

Wird nicht kontinuierlich hydriert, so empfiehlt sich die sogenannte "halbkontinuierlich" durchgeführte Hydrierung. Dabei wird der Katalysator in einem kleinen Teil des Fertigproduktes suspendiert und vorgelegt. Dann wird in das Reaktionsgefäß bei Reaktionstemperatur und Reaktionsdruck Butindiol-Lösung kontinuierlich in dem Maße, wie die Hydrierung stattfindet, eingeleitet. Auf diese Weise wird vermieden, daß Butindiol im zeitlichen Verlauf der Hydrierung in überhöhter Konzentration im Reaktionsvolumen auftritt.

Das im Reaktionsgemisch suspendierte Hydriermetall trennt man nach der Hydrierung, z. B. nach an sich üblichen physikalischen Trennmethoden, wie Zentrifugieren, Sedimentieren oder Filtrieren ab. Die Katalysatoren können erneut in den Reaktionsraum zurückgeführt werden. Sie erhalten ihre ursprüngliche Aktivität über sehr lange Reaktionszeiten bei.

Werden Nickelsalze verwendet, so bildet sich unter den Bedingungen des erfindungsgemäßen Verfahrens metallisches Nickel. Man trennt das im Reaktionsgemisch suspendierte Nickel nach der Hydrierung z. B. nach an sich üblichen physikalischen Trennmethoden, wie Zentrifugieren, Sedimentieren oder Filtrieren ab. Das so zuruckgewonnene Nickel kann z. B. durch Behandlung mit der entsprechenden organischen Säure, wie Essigsäure in eine Nickelsalz-Lösung überführt werden, die erneut für das erfindungsgemäße Verfahren herangezogen werden kann. Die dabei auftretenden Katalysatorverluste sind vernachlässigbar.

Nach dem neuen Verfahren, das im Gegensatz zu allen Erfahrungen bei Temperaturen arbeitet, die als ungeeignet für die Reaktion galten, erhält man die Alkandiole in außerordentlich hoher Reinheit. Die Selektivität beträgt praktisch 100 %. Dieser Befund ist überraschend, da man ein so vorteilhaftes Ergebnis z. B. nicht erhält, wenn man die Hydrierung der Alkindiole auf herkömmliche Art bei Temperaturen unter 200°C vornimmt. Das günstige Verfahrensergebnis konnte auch deshalb nicht erwartet werden, weil Alkine bekanntlich gegenüber hohen Temperaturen empfindlich sind und anzunehmen war, daß die in den Roh-Butindiol-Lösungen vorhandenen Nebenbestandteile (Aldehyde und Acetale) unter dem Einfluß so hoher Temperaturen polymere Rückstände bilden und damit unerwünschte Ablagerungen auf dem Katalysator verursachen würden. Erstaunlicherweise wird aber die Lebensdauer des Katalysators bei der erfindungsgemäßen Hydrierung nicht beeinträchtigt. So behält z. B. der unter den Reaktionsbedingungen verwendete Nickelkatalysator seine Aktivität bei, auch wenn er wiederholt für die gleiche Reaktion eingesetzt wird. Eine erwartungsgemäß erhöhte Butanolbildung und die damit verbundene Ausbeuteminderung trat bei hohen Reaktionstemperaturen entgegen dem allgemeinen Wissensstand nicht ein. Der Rückstandswert beträgt nur etwa 10 % der Menge, die der konventionelle Prozeß bei tieferer Temperatur liefert.

Das nach dem erfindungsgemäßen Verfahren erhältliche Butandiol zeichnet sich auch durch eine niedrige Polyesterfarbzahl aus, die normalerweise bei 40 APHA liegt. Nach den Angaben der US-PS-4, 213,000 wird ein Butandiol mit einer solchen Polyesterfarbzahl nur erhalten, wenn man in zwei Stufen hydriert, wobei man die Hydrierung der zweiten Stufe im sauren Bereich vornimmt.

**Beispiel 1**

Für die kontinuierliche Hydrierung von Butindiol-1,4 wurde eine wäßrige Butindiol-1,4-Rohlösung verwendet, die durch Umsetzung von Acetylen mit wäßrigem Formaldehyd (zur Herstellung siehe Ullmanns Enzyklopädie der technischen Chemie (1953), Band III, Seiten 109-119, Band IV, S. 754-757, DE-AS-2 421 407 und DE-OS-2 536 273) erhalten wurde. Sie bestand aus 0,6 Gew.-% Formaldehyd, 0,6 Gew.-% Formaldehyd-Polymeren, 39 Gew.-% Butin-2-diol-1,4, 55 bis 60 Gew.-% Wasser sowie aus 2 bis 4 Gew.-% nicht identifizierten Acetalen und undestillierbaren Anteilen.

Die Hydrierung wurde in einem senkrecht stehenden Reaktionsrohr mit 1.000 Volumenteilen Reaktionsinhalt durchgeführt. Das Verhältnis des Durchmessers zur Länge des Reaktionsrohres betrug 1 : 40. Das Reaktionsrohr enthielt keine Einbauten. Es wurde von unten nach oben mit Zulauf beschickt, der am oberen Ende in einen Hochdruckabscheider abfloß. Die Wasserstoffzufuhr erfolgte ebenfalls am unteren Ende des Reaktionsrohres. Der Wasserstoffdruck auf dem Hochdruckabscheider und dem Reaktionsrohr betrug 250 bar.

Aus der Gasphase des Hochdruckabscheiders wurden stündlich $10^4$ bis $10^6$ Normalvolumenteile Wasserstoff als Abgas entnommen. Zum Zulauf, der aus der oben genannten Ausgangslösung bestand, gab man so viel Nickelacetat, daß das wäßrige Gemisch, in dem sich das Nickelacetat löste, einen Nickelgehalt von 0,1 Gew.-% aufwies. Der Zulauf wurde auf 180°C vorgewärmt und in das Reaktionsrohr kontinuierlich eingepumpt. Die freiwerdende Hydrierwärme wurde mittels einer innen liegenden Rohrschlange nach außen über einen Wärmetauscher abgeführt (die Wärme kann mit gleich gutem Erfolg auch durch direkte Kühlung mittels eines im Kreise geführten Teilstromes des Reaktionsproduktes in einem außen liegenden Kühler abgeführt werden). Im Reaktionsrohr wurde eine mittlere Temperatur von 230 bis 250°C eingehalten. Dem Reaktionsrohr wurde die zu hydrierende Lösung mit einer Zulaufgeschwindigkeit von 150 Teilen pro Stunde zugeführt. Man erhielt stündlich 150 Teile eines Reaktionsaustrages, für dessen Zusammensetzung, wasserfrei gerechnet, die folgenden Bestandteile ermittelt wurden:

| | | |
|---|---|---|
| Methanol | 1,0 | Gew.-% |
| n-Butanol | 0,6 | Gew.-% |
| Hydroxybutyraldehyd | 0,01 | Gew.-% |
| Butandiol-1,4 | 97,9 | Gew.-% |
| Methylbutandiol | 0,2 | Gew.-% |
| Acetale | 0,01 | Gew.-% |
| Ethanol | 0,1 | Gew.-% |
| nicht flüchtige Anteile | 0,2 | Gew.-% |

Nach 20 Tagen Reaktionsdauer konnte die Frischzufuhr an Nickelacetat auf 10 % der ursprünglichen Konzentration gesenkt werden, ohne daß die Hydrierung beeinträchtigt wurde. Nach einigen Tagen Reaktionszeit erschien metallisches Nickel im Reaktionsaustrag. Dieses wurde mittels einer Filterkerze, die zwischen Reaktionsrohr und Hochdruckabscheider eingebaut war, abfiltriert. Nach weiteren 20 Tagen wurde der Reaktionsaustrag auf eine parallel geschaltete Metallfilterkerze umgeleitet und gelangte von dorthin in den Hochdruckabscheider. Nach Entfernen der ersten Metallfilterkerze wurde diese mit Eisessig ausgelaugt und das so abfiltrierte Nickel so in Nickelacetat umgewandelt. Die so gewonnene Nickelacetat-Lösung ließ sich, gegebenenfalls nach erfolgter Neutralisation mit z. B. Natriumcarbonat erneut für weitere Hydrierungen nach diesem Verfahren verwenden. Abgesehen von mechanischen Verlusten wurde kein Katalysatorverbrauch festgestellt.

**Beispiel 2**

Es wurde wie im Beispiel 1 beschrieben verfahren, wobei dem Reaktionsrohr stündlich 130 Teile einer 60 gew.-%-igen wäßrigen Lösung von Hexin-3-diol-2,5, die so viel Nickelacetat in gelöster Form enthielt, daß der Nickelgehalt in der Lösung 0,1 Gew.-% betrug, zugeführt wurde. Der gaschromatographisch untersuchte Reaktionsaustrag bestand (Wasser nicht berücksichtigt) zu 99,5 Gew.-% aus Hexandiol-2,5 und nur zu etwa 0,1 Gew.-% aus Hexanol-2.

**Beispiel 3**

In einem gerührten Druckbehälter mit einem Reaktionsvolumen von 5.000 Raumteilen wurden 500 Gew.-Teile einer rohen Butandiol-Lösung gegeben, die nach Beispiel 1 erhalten worden war. Außerdem wurden 25 Gew.-Teile Raney-Nickel in das Reaktionsgefäß gegeben. Bei 230°C und einem Gesamtdruck von 250 bar (Wasserstoff- und Dampfdruck des Reaktionssystems) wurde der Reaktionsapparat kontinuierlich mit stündlich 150 Gew.-Teilen der in Beispiel 1 als Ausgangslösung beschriebenen Butin-diol-1,4-Rohlösung beschickt. Dieser Zulauf gelangte nach Passieren einer im Reaktionsraum untergebrachten Rohrschlange mit einer Eingangstemperatur von 170°C in den Rührreaktor. Es setzte sofort eine stark exotherme Reaktion ein. Durch Wasserkühlung unter Druck in einer Innenschlange sorgte man für die Einhaltung einer Reaktionstemperatur von 230 bis 245°C. Nach 23 Stunden wurde der Zulauf abgestellt. Zur Vervollständigung der Umsetzung wurde noch 20 Minuten weiter hydriert. Nach Abkühlung des Reaktionsgemisches wurden 3.950 Teile eines Reaktionsaustrages erhalten, welcher, wasserfrei gerechnet, die in Beispiel 1 genannte Zusammensetzung aufwies.

Wird bei diesem Beispiel eine ähnliche Butindiol-1,4-Rohlösung mit einem Formaldehydgehalt von 2 Gew.-% eingesetzt, so erhält man einen Reaktionsaustrag mit ähnlicher Zusammensetzung, in dem der Methanolgehalt etwa 2 Gew.-% beträgt.

**0 177 912**

**Beispiel 4**

Beispiel 3 wurde wiederholt, wobei man jedoch anstelle von Raney-Nickel 100 Teile eines Katalysators verwendete, der durch Vermahlen eines für die Butindiol-Hydrierung gebräuchlichen Strängekatalysators (Zusammensetzung: 16,5 % Nickel, 5 % Kupfer und 0,7 % Mangan auf Kieselgel-Träger) erhalten wurde. Die Butindiol-Rohlösung wurde mit praktisch quantitativer Ausbeute umgesetzt. Auch in diesem Falle wurde eine Reaktionslösung erhalten, die der im Beispiel 1 beschriebenen Zusammensetzung entsprach.

**Patentansprüche**

1. Verfahren zur Herstellung von Alkandiolen von hoher Reinheit durch Hydrierung von Alkindiolen in wäßrigem Medium und in Gegenwart eines Hydrierkatalysators bei einem Wasserstoffpartialdruck von über 20 bar, dadurch gekennzeichnet, daß man die Hydrierung bei Temperaturen von über 200°C vornimmt.

2. Verfahren nach Anspruch 1, dadurch gekennzeichnet, daß man bei Temperaturen zwischen 210 und 280°C hydriert.

3. Verfahren nach Anspruch 1, dadurch gekennzeichnet, daß man die Hydrierung mit einem suspendierten Hickelkatalysator durchfuhrt.

4. Verfahren nach Anspruch 1, dadurch gekennzeichnet, daß man zu dem zu hydrierenden wäßrigen Gemisch ein wasserlösliches Nickelsalz einer organischen Säure mit mindestens 2 C-Atomen gibt und nach der Hydrierung aus dem Reaktionsgemisch metallisches Nickel mechanisch abtrennt.

5. Verfahren nach Anspruch 1, dadurch gekennzeichnet, daß man Butan-diol-1,4 aus Butin-2-diol-1,4 herstellt.

6. Verfahren nach Anspruch 1, dadurch gekennzeichnet, daß man bei Temperaturen von 230 bis 260°C und Wasserstoffpartialdrücken von 50 bis 250 bar hydriert.

7. Verfahren nach Anspruch 4, dadurch gekennzeichnet, daß man das abgetrennte Nickel durch Behandlung mit einer organischen Säure mit mindestens 2 C-Atomen in das Nickelsalz überführt und dieses direkt für die Hydrierung heranzieht.

8. Verfahren nach Anspruch 1, dadurch gekennzeichnet, daß man bei einem pH-Wert größer als 6 hydriert.

**Claims**

1. A process for the preparation of a very pure alkanediol by hydrogenating an alkyhediol in an aqueous medium and in the presence of a hydrogenation catalyst under a hydrogen partial pressure of above 20 bar, wherein the hydrogenation is carried out at above 200°C.

2. A process as claimed in claim 1, wherein hydrogenatrion is carried out at from 210 to 280°C.

3. A process as claimed in claim 1, wherein the hydrogenation is carried out using a suspended nickel catalyst.

4. A process as claimed in claim 1, wherein a watersoluble nickel salt of an organic acid of not less than 2 carbon atoms is added to the aqueous mixture to be hydrogenated, and metallic nickel is separated off mechanically from the reaction mixture when the hydrogenation is over.

5. A process as claimed in claim 1, wherein butane-1, 4-diol is prepared from but-2-yne-1, 4-diol.

6. A process as claimed in claim 1, wherein hydrogenation is carried out at from 230 to 260°C and under a hydrogen partial pressure of from 50 to 250 bar.

7. A process as claimed in claim 4, wherein the nickel separated off is treated with an organic acid of not less than 2 carbon atoms to convert it to the nickel salt, and the latter is used directly for the hydrogenation.

8. A process as claimed in claim 1, wherein hydrogenation is carried out at a pH greater than 6.

**Revendications**

1. Procédé de préparation d'alcan-diols de grande pureté par l'hydrogénation d'alcyn-diols en milieu aqueux, en présence d'un catalyseur d'hydrogénation et sous une pression partielle d'hydrogène supérieure à 20 bars, caractérisé en ce que l'hydrogénation est effectuée à des températures supérieures à 200°C.

2. Procédé suivant la revendication 1, caractérisé en ce que l'hydrogénation est réalisée entre 210 et 280°C.

3. Procédé suivant la revendication 1, caractérisé en ce que l'hydrogénation est effectuée en présence d'un catalyseur au nickel en suspension.

4. Procédé suivant la revendication 1, caractérisé en ce que l'on ajoute au mélage aqueu à hydrogéner un sel de nickel d'un acide organque avec au moins deux atomes de carbone, soluble dans l'eau, et du mélange réactionnel, on sépare après l'hydrogénation du nickel métallique par voie mécaique.

5. Procédé suivant la revendication 1, caractérisé en ce que l'on prépare du butane-diol-1,4 à partir du butyne-2 diol-1, 4.

6

6. Procédé suivant la revendication 1, caractérisé en ce que l'hydrogénation est réalisée à des températures comprises entre 230 et 260°C et avec des pressions partielles d'hydrogène de 50 à 250 bars.

7. Procédé suivant la revendication 4, caractérisé en ce que l'on trasforme le nickel récupéré à l'aide d'un acide orgaique avec au moins deux atomes de carbone en sel de nickel, qui est recyclé dans l'hydrogénation.

8. Procédé suivant la revendication 1, caractérisé en ce que l'hydrogénation est effectuée à pH supérieur à 6.